**Europäisches Patentamt**

**European Patent Office**

**Office européen des brevets**

(11) Publication number : **0 293 347 B1**

(12) **EUROPEAN PATENT SPECIFICATION**

(45) Date of publication of patent specification :
**10.06.92 Bulletin 92/24**

(21) Application number : **88850123.6**

(22) Date of filing : **08.01.85**

(51) Int. Cl.⁵ : **C07C 217/54,** A61K 31/135, A61K 9/00

(54) **Metoprolol succinate and pharmaceutical composition containing it.**

The file contains technical information
submitted after the application was filed and
not included in this specification

(30) Priority : **10.01.84 SE 8400085**

(43) Date of publication of application :
**30.11.88 Bulletin 88/48**

(45) Publication of the grant of the patent :
**10.06.92 Bulletin 92/24**

(84) Designated Contracting States :
**AT BE CH DE FR GB IT LI LU NL SE**

(56) References cited :
**DE-B- 2 106 209**
**GB-A- 1 492 647**
**US-A- 3 996 382**
**US-A- 4 145 442**

(60) Publication number of the earlier application in
accordance with Art. 76 EPC : **0 148 811**

(73) Proprietor : **Aktiebolaget Hässle**
**Kärragatan 5**
**S-431 83 Mölndal (SE)**

(72) Inventor : **Appelgren, Curt Henry**
**Benjaminssons väg PI 1461**
**S-434 00 Kungsbacka (SE)**
Inventor : **Eskilsson, Eva Christina**
**Körsbärsvägen 15**
**S-435 00 Mölnlycke (SE)**

(74) Representative : **Linderoth, Margareta et al**
**AB Astra Patent Department**
**S-151 85 Södertälje (SE)**

Note : Within nine months from the publication of the mention of the grant of the European patent, any
person may give notice to the European Patent Office of opposition to the European patent granted.
Notice of opposition shall be filed in a written reasoned statement. It shall not be deemed to have been
filed until the opposition fee has been paid (Art. 99(1) European patent convention).

## Description

The present invention relates to a new oral, therapeutically active compound, which can be released in the gastrointestianl tract below the upper part of the small intestine.

The object of the present invention is to obtain a therapeutically active compound, intended to be released close to or within the colon, and particularly such an active compound which is soluble in the pH range 1 to 8.

There exists a problem within pharmacy which is to be able to administer a therapeutically active compound as close as possible to the colon in order to thereby to eliminate the risk for acidic influence on the active compound by the gastric juice, or to prevent from irritation the ventricular mucous membrane due to a reflux, or to obtain a therapeutical effect in the lower part of the gastrointestinal tract.

As suitable active compounds have, i.a., previously been proposed propranolol, alprenolol, and metoprolol tartrate, quinidine sulfate, quinidine bisulphate, quinidine hydrochloride, furosemide, and 5-aminosalicylic acid, i.e. such weak bases or salts thereof, the pH of which is 1 to 8.

Known in the art is metoprolol hydrochloride (DE-B2-21 06 209). For a number of the free bases of beta-blocking agents other than metoprolol, a number of inorganic and organic acids, among them succinic acid, have been suggested as salt-forming acids (GB-A-1 492 647, US-A-3 996 382 and US-A-4 145 442).

It has now surprisingly been shown that it is advantageous to use, for this purpose, a previously unknown compound, viz. metoprolol succinate.

This compound can, in order to be administered orally, be treated in accordance with the method proposed in EP-A1-0 040 590. Herein it has been proposed an oral pharmaceutical composition comprising a core containing a therapeutically active compound, which core has been coated with a layer comprising 10 to 85 % by weight of an anionic polymer soluble at pH above 5.5, and 15 to 90 % by weight of a water-insoluble polymer selected from the group of quarternary ammonium-substituted acrylic polymers. From the point of view of flavour and/or identification, a flavored or coloured layer can optionally be applied outside the release-controlling layer. This is, however, not part of the present invention.

When dosing the ready-made product, a number of discrete coated particles/granules corresponding to a therapeutical dose unit of the actual therapeutic compound is administered.

When administering, in order to achieve a steady blood plasma level of the therapeutically active compound, a split dose unit of the therapeutically active compound provided with a coating according to the present invention can be administered together with some particles/granules which are not coated.

The particles are normally packed in small envelopes, tubular containers, or other capsules comprising a dose unit of a therapeutically active compound.

## Claims

### Claims for the following Contacting States : BE, CH, DE, FR, GB, IT, LI, LU, NL, SE

1. Metoprolol succinate.
2. A pharmaceutical composition, characterized in that the active compound is metoprolol succinate.

### Claims for the following Contacting State : AT

1. Use of metoprolol succinate in the preparation of a pharmaceutical formulation for release in the gastrointestinal tract below the upper part of the small intestine.
2. A process for the preparation of a pharmaceutical composition containing metoprolol succinate whereby metoprolol succinate is mixed with pharmaceutical carriers.

## Patentansprüche

### Patentansprüche für folgenden Vertragsstaaten : BE, CH, DE, FR, GB, IT, LI, LU, NL, SE

1. Metoprololsuccinat.
2. Pharmazeutische Zusammensetzung, dadurch gekennzeichnet, daß die aktive Verbindung Metoprololsuccinat ist.

### Patentansprüche für folgenden Vertragsstaat : AT

1. Verwendung von Metoprololsuccinat bei der Herstellung einer pharmazeutischen Formulierung zur Freisetzung im Gastrointestinaltrakt unter dem oberen Teil des Dünndarms.
2. Verfahren zur Herstellung einer Metoprololsuccinat enthaltenden pharmazeutischen Zusammensetzung, wobei Metoprololsuccinat mit pharmazeutischen Trägern gemischt wird.

## Revendications

### Revendications pour les Etats contractants suivants : BE, CH, DE, FR, GB, IT, LI, LU, NL, SE

1. Le succinate de métoprolol.

2. Composition pharmaceutique, caractérisée en ce que le composé actif est le succinate de métoprolol.

### Revendications pour l'Etat contractant suivant : AT

1. Utilisation du succinate de métoprolol dans la préparation d'une formulation pharmaceutique pour libération dans le tube digestif au-dessous de la partie supérieure de l'intestin grêle.

2. Procédé de préparation d'une composition pharmaceutique contenant du succinate de métoprolol, selon lequel on mélange le succinate de métoprolol avec des excipients, supports ou véhicules pharmaceutiques.